(19) Europäisches Patentamt
European Patent Office
Office européen des brevets

(11) **EP 0 966 234 B1**

(12) **EUROPÄISCHE PATENTSCHRIFT**

(45) Veröffentlichungstag und Bekanntmachung des Hinweises auf die Patenterteilung:
**01.10.2003 Patentblatt 2003/40**

(21) Anmeldenummer: **98910703.2**

(22) Anmeldetag: **19.02.1998**

(51) Int Cl.[7]: **A61B 19/00**, A61B 5/06

(86) Internationale Anmeldenummer:
**PCT/EP98/00940**

(87) Internationale Veröffentlichungsnummer:
**WO 98/037826 (03.09.1998 Gazette 1998/35)**

(54) **MARKER ZUR BESTIMMUNG SEINER POSITION IN EINEM HOHLRAUM INNERHALB DES ORGANISMUS EINES LEBEWESENS**

MARKER FOR DETERMINING ITS POSITION IN A CAVITY INSIDE THE ORGANISM OF A LIVING BEING

MARQUEUR POUR DETERMINER SA POSITION DANS UNE CAVITE SITUEE DANS L'ORGANISME D'UN ETRE VIVANT

(84) Benannte Vertragsstaaten:
**AT BE CH DK ES FI FR GB GR IE IT LI LU MC NL PT SE**

(30) Priorität: **26.02.1997 DE 19707556**

(43) Veröffentlichungstag der Anmeldung:
**29.12.1999 Patentblatt 1999/52**

(73) Patentinhaber:
• **Carl Zeiss Meditec AG
07745 Jena (DE)**
• **INSTITUT FÜR PHYSIKALISCHE HOCHTECHNOLOGIE E.V.
07745 Jena (DE)**

(72) Erfinder: **ANDRÄ, Wilfried
D-07749 Jena (DE)**

(74) Vertreter: **Bock, Gerhard
Patentanwälte
Bock & Bieber GbR
Winzerlaer Strasse 10
07745 Jena (DE)**

(56) Entgegenhaltungen:
**WO-A-97/09640          DE-A- 3 940 260**

• **"NEUE METHODEN ZUR BESTIMMUNG DER GASTROINTESTINALEN TRANSITZEIT" BIOMEDIZINISCHE TECHNIK, Bd. 35, Nr. 7 / 08, 1.Juli 1990, Seiten 179-180, XP000168176**

**Beschreibung**

Die Erfindung betrifft einen Marker, dessen Position in einem Hohlraum innerhalb des Organismus eines Lebewesens bestimmbar sein soll, insbesondere zur Ermittlung lokaler Passage-Geschwindigkeiten des Markers durch den Magen-Darm-Trakt, bevorzugt im Dünndarmbereich. Der Marker soll insbesondere innerhalb eines Verfahrens und einer Anordnung gemäß WO 97/09640 Verwendung finden.

Es sind medizinische Untersuchungen bekannt, bei denen eine wiederholte Bestimmung der lokalen Passage-Geschwindigkeit eines Markers durch den Magen-Darm-Trakt erforderlich ist. Das ist z.B. bei chronisch entzündlichen Darmerkrankungen, wie etwa Morbus Chron, funktionelle Magen-Darm-Erkrankungen und physiologische Untersuchungen des Magen-Darm-Traktes der Fall.

Die übliche Diagnose-Technik, wie Röntgen unter Verwendung von Kontrastbrei, darf in solchen Fällen wegen der Strahlenbelastung nicht angewendet werden. Dasselbe gilt für szintigraphische Methoden.

Bekannte Techniken, bei denen die Strahlenbelastung vermieden wird, sind die Kernspin-Tomographie [M. Reiser, W. Semmler(Hrsg) "Magnetresonanztomographie", Springer-Verlag, Berlin/Heidelberg, 1992], die Sonographie [M. Amend, C. Jakobeit, L. Greiner, Verdauungskrankheiten 13 (1995), Heft 1, S. 21], der Einsatz von Metall-Detektoren [K. Ewe, Therapiewoche 41 (1991), S. 77], der induktive Nachweis weichmagnetischer Tracer [Y. Benmair, B. Fischel, E. H. Frei, T. Gilat, The American Journal of Gastroenterology 68 (1977), S. 470] und das Orten von Dauermagnet-Markern [L. Trahms, R. Stehr, J. Wedemeyer, W. Weitschies, Biomedizinische Technik 35 (1990) S. 158].

Die Kernspin-Tomographie oder Magnet-Resonanz ist ein aufwendiges und teures Verfahren, das für häufig zu wiederholende Untersuchungen nicht geeignet und zur Ermittlung einer lokalen Passage-Geschwindigkeit, die einen zeitlichen Abstand aufeinanderfolgender Positionsbestimmungen von Markern in der Größenordnung von 10 s erforderlich macht, zu langsam ist [K. Fitzgerald, IEEE Spectrum 27 (1990) S. 52].

Sonographische Untersuchungen wurden bisher nicht zur Bestimmung der lokalen Passage-Geschwindigkeit, sondern nur zum Messen pauschaler Transitzeiten größerer Abschnitte des Magen-Darm-Traktes eingesetzt. [M. Amend, C. Jakobeit, L. Greiner, Verdauungskrankheiten 13 (1995), Heft 1, S. 21]. Der Grund liegt darin, daß Luftvolumina im Bauchraum von Ultraschall nicht durchdrungen werden, was zu einer fehlerhaften Positionsbestimmung von Markern führen würde. Diese Fehler könnten zwar herabgesetzt werden, wenn der Darm vollständig mit einer Flüssigkeit gefüllt würde; wegen der dadurch veränderten Peristaltik ist aber ein gefüllter Darm zur Diagnose nicht geeignet.

Mit Metalldetektoren kann die Position von Metallteilchen bestimmt werden. Die laterale Genauigkeit bei der Positionsbestimmung nimmt jedoch mit dem Abstand von der Körperoberfläche ab und wird bei einem Abstand von > 10 cm schlechter als 1 cm [K. Ewe, Therapiewoche 41 (1991), S. 77]. Über die Genauigkeit einer Tiefenmessung wird in dieser Arbeit nichts berichtet. Da sie jedoch grundsätzlich schlechter als die laterale Genauigkeit ist, reicht dieses Verfahren für die Messung der lokalen Passage-Geschwindigkeit nicht aus.

Die Genauigkeit der Positionsbestimmung bei einer induktiven Messung weichmagnetischer Tracer genügt zur Untersuchung der zeitlichen Abnahme des Mageninhaltes an weichmagnetischem Brei mit einem Anfangsvolumen von über 100 $cm^3$ [Y. Benmair, B. Fischel, E. H. Frei, T. Gilat, The American Journal of Gastroenterology 68 (1977), S. 170]. Eine Messung der lokalen Passage-Geschwindigkeit im Darm ist damit jedoch nicht möglich, da das große Probenvolumen sich beim Durchlaufen des Darms unkontrolliert verteilt. Das Probenvolumen kann andrerseits aber auch nicht wesentlich verkleinert werden, da dann das vom Tracer stammende Sekundärmagnetfeld so klein wird, daß es selbst bei extrem guter Kompensation eines während der Messung angelegten Primärmagnetfeldes nicht mehr von dessen Restsignal getrennt werden kann.

Weiterhin ist bekannt, permanent magnetische Marker aufzumagnetisieren, bevor sie einem Patienten verabreicht werden [W. Weitschies, J. Wedemeyer, R. Stehr, L. Trahms, IEEE Trans. Biomed. Eng. 41 (1994) S. 192], [DE 39 40 260]. Die Positionsbestimmung der Marker aus ihrem Sekundärmagnetfeld wird hierbei allerdings durch Störfelder (z.B. das Erdmagnetfeld) so stark beeinflußt, daß die Messungen in einer Spezialkammer mit extremer magnetischer Abschirmung ausgeführt werden müssen. Selbst dann ist aber dieses Verfahren nicht für die Bestimmung der lokalen Passage-Geschwindigkeit im gesamten Magen-Darm-Trakt geeignet, da wegen der Transversal- und Rotationsbewegungen der Marker die Positionsbestimmung lediglich im Magen oder im Dickdarm möglich ist und selbst in diesen Bereichen mit einer relativ hohen Verweildauer des Markers nur mit einer ungenügenden Genauigkeit.

In WO 97/09640 wurde eine Anordnung und ein Verfahren zur Bestimmung der Position eines magnetisierbaren Markers vorgeschlagen, wobei der Marker aus einem halbharten Magnetmaterial mit einer Koerzitivfeldstärke im Bereich von $10^4$ bis $10^5$ A/m bestehen soll und der Marker kugelsymmetrisch ausgebildet ist. Der Marker soll gemäß dieser Schrift weiterhin aus einem isotropen Magnetmaterial bestehen, das eine relative Remanenz von vorzugsweise > 0,8 besitzt, wobei der Marker im wesentlichen aus $\gamma$-$Fe_2O_3$ und/oder $Fe_3O_4$ gefertigt ist. Ein Nachteil der dort vorgeschlagenen Markerausbildung besteht darin, daß das erforderliche Primärmagnetfeld zur Ummagnetisierung des Markers recht erheblich ist.

Der Erfindung liegt die Aufgabe zugrunde, einen Marker zu schaffen, der von einer Magnetspulenanordnung erfaßbar ist, wobei dessen Magnetisierung mit relativ kleinen Feldstärken bei einem Wechsel eines impulsförmigen Stromflusses in der Magnetspulenanordnung umkehrbar ist.

Die Aufgabe wird durch die Kennzeichen des Patentanspruchs 1 gelöst. Gemäß der Erfindung ist der magnetisierbare Marker aus zwei Teilen gefertigt, von denen das erste ein im wesentlichen kugelförmig ausgebildetes Teil ist, das der Aufnahme eines magnetisierbaren Materials oder Körpers dient. Dieses erste Teil wird vom zweiten Teil derart aufgenommen, daß es allseitig drehbar schwimmend im zweiten Teil gelagert ist. Die Lage des äußeren zweiten Teils wird bei der Passage durch den Magen-Darm-Trakt von Kräften bestimmt, die der Speisebrei, die Darmwandung und die Peristaltik ausüben. Dagegen ist die Orientierung des inneren, ersten im wesentlichen kugelförmigen Teils durch das auf den Magneten wirkende Feld, z.B. Erdmagnetfeld, bestimmt. Diese Orientierung kann durch ein extern angelegtes Feld, das stärker als das Erdmagnetfeld oder ein zufällig vorhandenes Labormagnetfeld ist, willkürlich eingestellt werden. Dazu sind bereits Magnetfeldstärken von ca. 80 A/m ausreichend, die sehr einfach mit stromdurchflossenen Spulen oder Dauermagneten außerhalb eines Patientenkörpers erzeugt werden können.

Um bei Einsatz des erfindungsgemäßen Markers, bspw. in einer Anordnung nach WO 97/09640, den Einfluß äußerer Störfelder zu eliminieren, wird das magnetische Moment des ersten Teils durch ein kurzzeitig angelegtes Feld $H_{ext}$ in eine gewünschte Orientierung, z.B. senkrecht zur Ebene des Patientenrückens, gedreht. Danach wird das angelegte Feld kurzzeitig abgeschaltet und unmittelbar danach, so schnell wie möglich, das Markerfeld von vorgesehenen Sensoren gemessen. Nach dieser Messung wird das Feld $H_{ext}$ in der entgegengesetzten Richtung kurzzeitig angelegt und obiger Vorgang wiederholt. Durch die Bildung der Differenz beider gemessenen Markerfelder werden alle Störfeldeinflüsse eliminiert, die sich zwischen beiden Messungen nicht stark geändert haben. Die Zeiten, die für die Ausrichtung im extern angelegten Magnetfeld und für die Rückdrehung in Richtung des Erdoder Laborfeldes erforderlich sind, hängen u.a. vom remanenten magnetischen Moment genannten ersten Teils und von der Viskosität der Flüssigkeit ab, in der das erste Teil gelagert ist. Eine einfache, mit üblichen Fachwissen ausgebildete elektronische Schaltung reicht aus, um das Magnetfeld des Markers innerhalb einer Zeit von wenigen Zehntel Sekunden nach Abschalten des externen magnetischen Feldes $H_{ext}$ zu messen. Damit der genannte erste Teil während dieser Messung sich praktisch noch im ausgerichteten Zustand befindet, ist eine Rückdreh-Zeit $\Delta t_R$ von etwa 10 s oder mehr ausreichend. Um die für das Ausrichten durch $H_{ext}$ erforderliche Leistung gering zu halten, sollte die Ausricht-Zeit $\Delta t_A$ etwa 1 s nicht übersteigen. Die genannten Richtwerte von $\Delta t_R$ und $\Delta t_A$ können bei Verwendung handelsüblicher Dauermagnete mit Flüssigkeiten erreicht werden, die etwa die Viskosität von Wasser besitzen oder bis zu einem Faktor 100 übertreffen. Außerdem liegt die Beeinflussung beider Zeiten $\Delta t_R$ und $\Delta t_A$ durch eine entsprechende Formgebung der Oberflächen des ersten und/oder zweiten Teils und einer Variation des Abstandes der Oberflächen zwischen dem ersten und zweiten Teil im Rahmen der Erfindung.

Die Erfindung soll nachstehend anhand von Ausführungsbeispielen näher erläutert werden. Es zeigen:

Fig. 1 die Positionierung eines Patienten innerhalb einer möglichen Anordnung gemäß dem Stand der Technik,
Fig. 2 einen Schnitt durch einen Marker gemäß vorliegender Erfindung,
Fig. 2a einen Schnitt durch einen Marker mit einer modifizierten Oberflächengestaltung des ersten Teils und
Fig. 2b einen Schnitt durch einen Marker, der mit zwei ersten Teilen versehen ist.

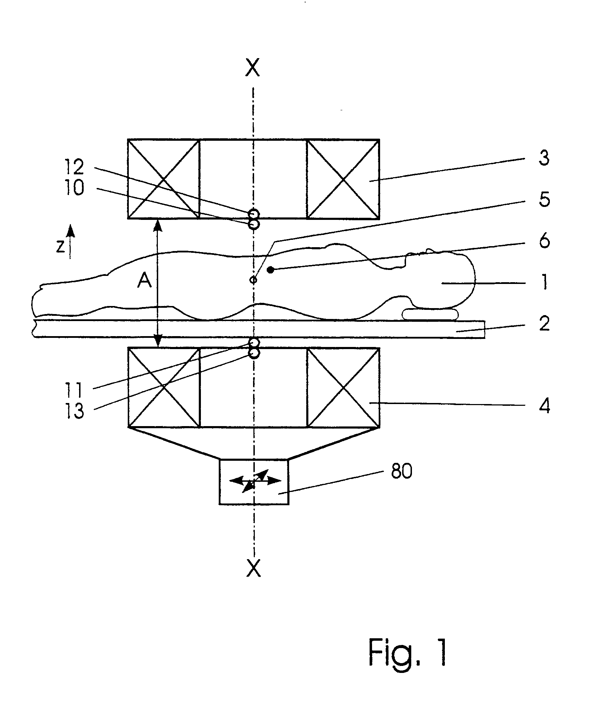

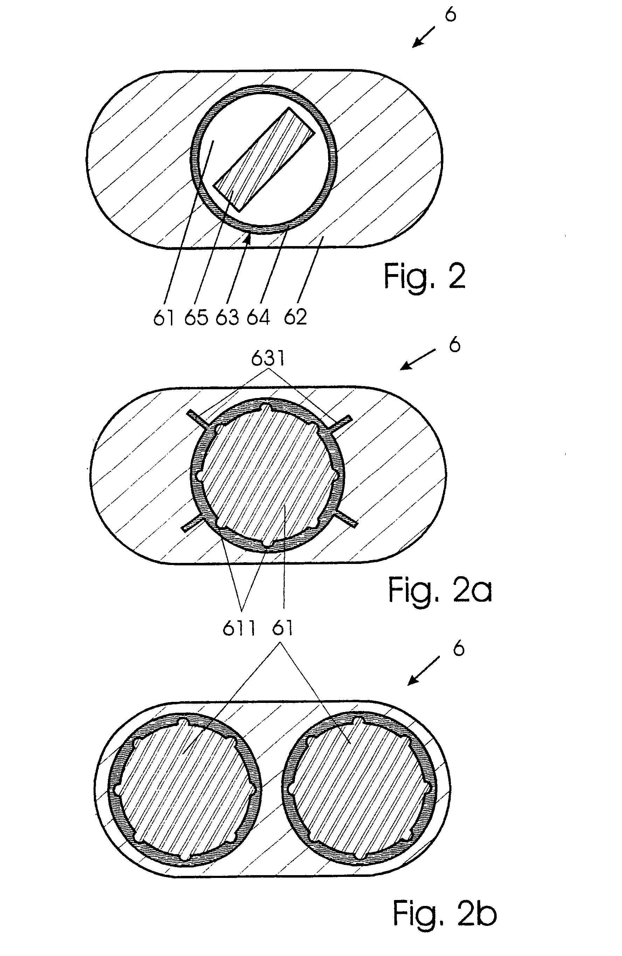

In Figur 1 liegt ein Patient 1, dessen Magen-Darm-Trakt untersucht werden soll, auf einer nichtmetallischen unmagnetischen Auflage 2. Zwei identische Spulen 3 und 4 sind mit ihren Spulenachsen auf einer gemeinsamen Achse X-X in einer lichten Weite A in der Größenordnung von 30 cm beabstandet angeordnet. Mittels eines nicht näher bezeichneten Generators werden die Spulen 3, 4 mit impulsförmigen Strömen beaufschlagt. Im geometrischen Zentrum 5 wird dabei ein maximales Primärmagnetfeld, je nach gewählter Stromimpulsfrequenz bspw. in einem Zeitabstand von 1 s erzeugt. Dem Patienten 1 wurde oral ein in Figur 2 näher beschriebener Marker 6 verabreicht.

Die Spulen 3, 4 sind jeweils mit anisotropen magnetoresistiven Magnetfeldsensoren 10, 11, 12, 13 in eine starre Verbindung gebracht. Dabei sind die Magnetfeldsensoren 10, 11 so ausgelegt, daß sie der ausschließlichen Detektion einer parallel zur Achse X-X verlaufenden Komponente $H^{\parallel}$ des vom Marker ausgehenden Sekundärmagnetfeldes und die Magnetfeldsensoren 12, 13 einer senkrecht dazu verlaufenden Radialkomponente $H^r$ genannten Sekundärmagnetfeldes dienen. Befindet sich der Marker 6 im Zentrum der gemeinsamen Spulenachse X-X, ist die Magnetfeldkomponente $H^{\parallel}$ ermittelbar, während die dazu senkrecht verlaufende Magnetfeldkomponente $H^r$ Null ist. Werden die Magnetfeldsensoren jeweils in vorgesehenen zeitlichen Pausen $t_1$ zwischen zwei Primärfeldimpulsen mit einem zeitlichen Abstand $t_2$ getriggert eingeschaltet, dann liefern die in Reihe geschalteten Radialsensoren 12, 13 nach einer radialen Markerverschiebung von bspw. r = 1 cm eine impulsförmige Wechselspannung. Die im Beispiel gemeinsame Spulenachse X-X wird nun mit Hilfe von Mitteln zur relativen Nachführung, die am einfachsten in Form eines mit den Spulen 3 und 4 in Verbindung stehenden Kreuztisches 80 ausgebildet sein können, solange verschoben, bis dieses Signal verschwunden ist. Ist dies der Fall, befindet sich die Spulenachse wieder im Markerzentrum. Ihre diesbezüglich eingenommene Lage wird mit einem nicht näher bezeichneten Ortssensor erfaßt und einer Speicher- und Auswerteeinheit zugeführt.

Nach einer Axialverschiebung des Markers 6 aus dem Zentrum 5 in z-Richtung werden von den beiden Axialsensoren 10, 11 impulsförmige Wechselspannungen erzeugt. Diese der jeweiligen vertikalen Markerposition zugeordneten Signale werden ebenfalls der nicht dargestellten Speicher- und Auswerteeinheit zugeführt. Somit steht für jede aktuelle Markerposition die erforderliche Anzahl von Meßwerten zur Verfügung, die seine aktuelle räumliche Lage im Magen-Darm-Trakt eindeutig beschreibt. Die auf die beschriebene Weise nacheinander gewonnenen Markerpositionen bilden eine Punktkette, die den Weg des Markers repräsentiert. Der Quotient aus dem Abstand benachbarter Punkte und dem Zeitintervall zwischen diesen Punkten steht dabei als Maß für die zu ermittelnde lokale Passage-Geschwindigkeit des Markers 6. All die gewonnenen Meßdaten lassen sich nach entsprechender Auswertung und Kalibrierung während oder nach der Untersuchung der Markerbewegung im Patienten auf einem Monitor dreidimensional zur Anzeige bringen.

In Figur 2 ist ein Marker dargestellt, der aus einem ersten im wesentlichen kugelförmigen Teil 61 besteht. Dieses Teil 61 ist mit einem magnetisierbaren Material verfüllt oder enthält einen magnetisierbaren Körper 65. Weiterhin ist ein zweites Teil 62 vorgesehen, dem eine der Oberflächenform des ersten Teils angepaßte, im dargestellten Beispiel ebenfalls kugelförmige Aufnahme 63 gegeben ist. Diese Aufnahme 63 umfaßt das erste Teil 61 allseitig, wobei die Aufnahme 63 mit einer Flüssigkeit 64 geringer Viskosität verfüllt ist, wodurch eine allseitige Drehmöglichkeit des ersten Teils 61 innerhalb der Aufnahme 63 geschaffen ist. Dem zweiten Teil 62 kann ebenfalls eine kugelförmige äußere Gestalt gegeben sein, bevorzugt im Rahmen des Verwendungszwecks des Markers ist ihm jedoch eine medikamentenkapselähnliche äußere Gestalt gegeben.

Der Vorteil eines derart ausgebildeten Markers besteht darin, daß er in einem starken Feld außerhalb des Patientenkörpers aufmagnetisiert werden kann, wodurch er zu einer Quelle eines wesentlich stärkeren Sekundärmagnetfeldes (ca. 10fach stärker gegenüber eines in WO 97/09640 eingesetzten Markers) wird und somit auch größere Sensorsignale erzeugt. Das nach Fig. 1 erzeugte Primärmagnetfeld braucht die Magnetisierung des Markers nicht mehr umzudrehen, sondern nur noch das Teil 61 zu drehen. Dazu genügt ein Feld, das maximal ein Zehnfaches der Erdmagnetfeldstärke beträgt, während nach WO 97/09640 etwa die 1000fache Erdmagnetfeldstärke benötigt wird. Die nach WO 97/09640 vorgesehenen Spulen können erheblich leichter ausgeführt werden und benötigen nur etwa 1% der dort erforderlichen elektrischen Leistung.

In Figur 2a ist ein Schnitt durch einen Marker 6 dargestellt, bei dem dem ersten Teil 61 eine mit Erhebungen 611 versehene Oberflächengestaltung gegeben ist. Ebenso liegt es im Rahmen der Erfindung, das erste Teil 61 mit Erhebungen 611 und/oder nicht näher dargestellten Einbuchtungen zu versehen, als auch die Oberfläche der Aufnahme 63 mit Einbuchtungen 631 zu versehen, von denen in Fig. 2a schematisch vier angedeutet sind. Für den Fall einer erhebungsfreien Oberfläche des ersten Teils 61 kann die Oberfläche der Aufnahme 63 mit Erhebungen ausgestattet sein. Alle zu Fig. 2a bisher beschriebene Ausführungsmöglichkeiten, als auch eine definierte Einstellung der Viskosität der Flüssigkeit 64, bspw. durch Mischung zweier Komponenten, insbesondere von Ölen, bevorzugt Speiseöle und eine gezielte Anpassung des Abstandes zwischen der Oberfläche des ersten Teils 61 zur Oberfläche der Aufnahme 63 dienen der gezielten Einstellung und Vorgabe der Zeiten $\Delta t_R$ und $\Delta t_A$. Weiterhin liegt es im Rahmen der Erfindung, den ersten Teil 61 vollständig mit einem magnetisierbaren Material zu verfüllen, wie in Fig. 2a angedeutet. Ebenso kann es vorteilhaft sein, die Verfüllung des Teils 61 so vorzunehmen, daß der Masseschwerpunkt des Teils 61 außerhalb seines geometrischen Mittelpunktes liegt.

Schließlich liegt es ebenfalls im Rahmen der Erfindung, im zweiten Teil 62 des Markers 6 mehrere erste Teile 61 vorzusehen, von denen in Fig. 2b zwei dargestellt sind. Dies führt bspw. zu einer Erhöhung des vom Marker 6 erhaltbaren Signals.

Alle in der Beschreibung, den nachfolgenden Ansprüchen und der Zeichnung dargestellten Merkmale können sowohl einzeln als auch in beliebiger Kombination miteinander erfindungswesentlich sein.

**Patentansprüche**

1. Marker zur Bestimmung seiner Position in einem Hohlraum innerhalb des Organismus eines Lebewesens, wobei der Marker aus wenigstens einem ersten, im wesentlichen kugelförmigen Teil (61), welches ein magnetisierbares Material enthält, besteht, **dadurch gekennzeichnet, daß** das erste Teil (61) von einem zweiten Teil (62), dem eine, der Oberflächenform des ersten Teils angepaßte, im wesentlichen kugelförmige Aufnahme (63) gegeben ist, allseitig umfaßt ist, wobei die Aufnahme (63) mit einer Flüssigkeit (64) geringer Viskosität verfüllt ist.

2. Marker nach Anspruch 1, **dadurch gekennzeichnet, daß** dem zweiten Teil (62) eine medikamentenkapselähnliche äußere Gestalt gegeben ist.

3. Marker nach Anspruch 1, **dadurch gekennzeich-**

**net, daß** die Oberfläche des ersten Teils (61) mit Erhebungen (611) und/oder Einbuchtungen versehen ist.

4. Marker nach Anspruch 1, **dadurch gekennzeichnet, daß** die Oberfläche der Aufnahme (63) mit Erhebungen und/oder Einbuchtungen (631) versehen ist.

5. Marker nach Anspruch 1 oder 3, **dadurch gekennzeichnet, daß** das erste Teil (61) mit einem magnetisierbaren Material derart verfüllt ist, daß der Masseschwerpunkt des Teils (61) außerhalb seines geometrischen Mittelpunktes liegt.

6. Marker nach Anspruch 1, **dadurch gekennzeichnet, daß** die Viskosität der Flüssigkeit (64) durch eine Mischung unterschiedlich viskoser Komponenten einstellbar ist.

7. Marker nach Anspruch 1 oder 6, **dadurch gekennzeichnet, daß** für die Flüssigkeit (64) ein oder mehrere Öle eingesetzt sind.

8. Marker nach Anspruch 7, **dadurch gekennzeichnet, daß** für die ein oder mehreren Öle ein oder mehrere Speiseöle gewählt sind.

**Claims**

1. Marker for determining the position of the same in a cavity inside the organism of a living being, said marker consists of at least one first, substantially spherical part (61) containing a magnetizable material, **characterized in that** said one first part (61) is enveloped on all sides by a second part (62) which is provided with a substantially spherically shaped receptacle (63) , adapted to the shape of the surface of said first part, wherein said receptacle (63) is filled with a liquid (64) of low viscosity.

2. Marker as claimed in claim 1, **characterized in that** said second part (62) is given an external shape similar to a drug capsule.

3. Marker as claimed in claim 1, **characterized in that** the surface of said first part (61) is provided with elevations (611) and/or recesses.

4. Marker as claimed in claim 1, **characterized in that** the surface of said receptacle (61) is provided with elevations and/or recesses (631).

5. Marker as claimed in claim 1 or 3, **characterized in that** said first part (61) is filled with a magnetizable material in such a manner that the center of mass of said part (61) lies outside of the geometric center of the same.

6. Marker as claimed in claim 1, **characterized in that** the viscosity of the liquid (64) is adjustable by a mixture from differently viscous components.

7. Marker as claimed in claim 1 or 6, **characterized in that** at least one oil is employed as said liquid (64).

8. Marker as claimed in claim 7, **characterized in that** at least one edible oil is employed as said at least one oil.

**Revendications**

1. Le marqueur destiné à localiser sa position dans une cavité située à l'intérieur d'un organisme vivant et comprenant au moins un premier module de forme quasiment sphérique (61), réalisé dans un matériau magnétisable, est **caractérisé en ce que** le deuxième module (62) doté d'un logement (63) et ayant également une forme quasiment sphérique, dont la surface est adaptée au premier module, embrasse complètement le premier module (61), le logement (63) étant rempli d'un fluide (64) de faible viscosité.

2. Le marqueur selon la revendication 1 est **caractérisé en ce que** la forme extérieure du deuxième module (62) rappelle une gélule de médicament.

3. Le marqueur selon la revendication 1 est **caractérisé en ce que** la surface du premier module (61) est doté de bosses (611) et/ou de zones creuses.

4. Le marqueur selon la revendication 1 est **caractérisé en ce que** la surface du logement (63) est pourvue de bosses et/ou de zones creuses (631).

5. Le marquer selon les revendications 1 ou 3 est **caractérisé en ce que** le premier module (61) est rempli d'un matériau magnétisable de sorte que le point de masse du module (61) se trouve situé en dehors de son centre géométrique.

6. Le marqueur selon la revendication 1 est **caractérisé en ce que** la viscosité du fluide (64) est réglable en mélangeant des composantes aux viscosités différentes.

7. Le marqueur suivant la revendication 1 ou 6 est **caractérisé en ce que** sont utilisés comme fluide (64) une ou plusieurs huiles.

8. Le marqueur suivant la revendication 7 est **caractérisé en ce que** le ou les huiles sont de préférence une ou plusieurs huiles alimentaires.

Fig. 1

Fig. 2

Fig. 2a

Fig. 2b